# EUROPEAN PATENT APPLICATION

(11) **EP 4 775 678 A1**
(43) Date of publication of application: **15.07.2026**
(21) Application number: 24873035.0
(22) Date of filing: 27.09.2024
(51) Int. Cl.: C12N 15/77, C12N 9/88, C12P 13/08, C12P 13/06, C12R 1/15

(54) **MICROORGANISM INCORPORATING THREONINE DEHYDRATASE DERIVED FROM ARABIDOPSIS THALIANA AND METHOD FOR PRODUCING BRANCHED-CHAIN AMINO ACID USING SAME**

(30) Priority: 27.09.2023 KR 20230130426
(71) Applicant: CJ Cheiljedang Corporation, Seoul 04560 (KR)
(72) Inventor: YUN, Hyojin, Seoul 04560 (KR); OH, Haena, Seoul 04560 (KR); KIM, Seon Hye, Seoul 04560 (KR); SEO, Chang Il, Seoul 04560 (KR); SHIM, Jihyun, Seoul 04560 (KR); LEE, Hyein, Seoul 04560 (KR)
(74) Representative: Meissner Bolte Partnerschaft mbB
(86) International application number: PCT/KR2024/014718
(87) International publication number: WO 2025/071328

(57) **Abstract**

The present disclosure relates to: a microorganism of the genus *Corynebacterium,* into which a threonine dehydratase derived from *Arabidopsis thaliana* is introduced; a method for producing a branched-chain amino acid, comprising culturing the microorganism in a medium; a method for enhancing the production of a branched-chain amino acid, comprising culturing the microorganism in a medium; a composition for producing a branched-chain amino acid, comprising the microorganism, a culture of the microorganism, a fermentation product of the microorganism, or a combination of at least two thereof; and a use of the microorganism for producing a branched-chain amino acid.

## Description

### [Technical Field]

The present disclosure relates to a microorganism of the genus *Corynebacterium,* into which a threonine dehydratase derived from *Arabidopsis thaliana* is introduced; a method for producing a branched-chain amino acid, comprising culturing the microorganism in a medium; a method for enhancing the production of a branched-chain amino acid, comprising culturing the microorganism in a medium; a composition for producing a branched-chain amino acid, comprising the microorganism, a culture of the microorganism, a fermentation product of the microorganism, or a combination of at least two thereof; and a use of the microorganism for producing a branched-chain amino acid.

### [Background Art]

L-amino acids are the fundamental building blocks of proteins and are used as important materials for pharmaceutical raw materials, food additives, animal feed, nutritional supplements, insecticides, and fungicides. In particular, branched-chain amino acids (BCAAs) refer to L-valine, L-leucine, and L-isoleucine, which are essential amino acids, and the branched-chain amino acids are known to have antioxidant effects and effects of directly promoting protein synthesis in muscle cells.

Meanwhile, production of branched-chain amino acids using microorganisms is typically performed through a microorganism of the genus *Escherichia* or a microorganism of the genus *Corynebacterium.* For example, L-valine is known to be biosynthesized from pyruvic acid, using 2-oxoisovalerate as a precursor, through several steps but industrial-scale mass production of branched-chain amino acids through microorganisms is difficult to achieve.

In methods for mass production of L-valine using microorganisms, a method of blocking the L-isoleucine biosynthetic pathway is known. However, when the *ilvA* gene encoding L-threonine dehydratase in the L-isoleucine biosynthetic pathway is deleted, there lies a problem in that growth is limited due to requirement for L-isoleucine.

Accordingly, there remains a need for research to enhance branched-chain amino acid production efficiency while maintaining the rate of sugar consumption.

### [Disclosure]

### [Technical Problem]

The present disclosure intends to provide a microorganism of the genus *Corynebacterium,* into which a threonine dehydratase derived from *Arabidopsis thaliana* is introduced, and a use thereof.

### [Technical Solution]

One aspect of the present disclosure provides a microorganism of the genus *Corynebacterium,* into which a threonine dehydratase derived from *Arabidopsis thaliana* is introduced.

In one specific embodiment, the threonine dehydratase derived from *Arabidopsis thaliana* may comprise an amino acid sequence having at least 75% identity to SEQ ID NO: 15.

In another specific embodiment, the threonine dehydratase derived from *Arabidopsis thaliana* may be encoded by an *ilvA* gene.

In still another specific embodiment, the *ilvA* gene derived from *Arabidopsis thaliana* may comprise a base sequence having at least 75% identity to SEQ ID NO: 16.

As a microorganism according to any one of the foregoing specific embodiments, the microorganism may be one in which endogenous threonine dehydratase activity is further deleted.

As a microorganism according to any one of the foregoing specific embodiments, the microorganism of the genus *Corynebacterium* may be *Corynebacterium glutamicum.*

As a microorganism according to any one of the foregoing specific embodiments, the microorganism may have the ability to produce a branched-chain amino acid.

As a microorganism according to any one of the foregoing specific embodiments, the branched-chain amino acid may be L-valine or L-leucine.

As a microorganism according to any one of the foregoing specific embodiments, the microorganism may have enhanced ability to produce a branched-chain amino acid compared to a non-modified microorganism.

Another aspect of the present disclosure provides a method for producing a branched-chain amino acid, comprising culturing a microorganism of the genus *Corynebacterium,* into which a threonine dehydratase derived from *Arabidopsis thaliana* is introduced, in a medium.

In one specific embodiment, the method may further comprise recovering a branched-chain amino acid in the cultured microorganism, a culture of the microorganism, a fermentation product of the microorganism, or the medium after cultivation.

Still another aspect of the present disclosure provides a method for enhancing the production of a branched-chain amino acid, comprising culturing a microorganism of the genus *Corynebacterium,* into which a threonine dehydratase derived from *Arabidopsis thaliana* is introduced, in a medium.

Still another aspect of the present disclosure provides a composition for producing a branched-chain amino acid, comprising: a microorganism of the genus *Corynebacterium,* into which a threonine dehydratase derived from *Arabidopsis thaliana* is introduced; a culture of the microorganism; a fermentation product of the microorganism; or a combination of at least two thereof.

Still another aspect of the present disclosure provides a use of a microorganism of the genus *Corynebacterium,* into which a threonine dehydratase derived from *Arabidopsis thaliana* is introduced, for producing a branched-chain amino acid.

### [Advantageous Effects]

The microorganism of the genus *Corynebacterium* of the present disclosure, into which a threonine dehydratase derived from *Arabidopsis thaliana* is introduced, can produce a branched-chain amino acid at a high yield while maintaining the rate of sugar consumption, and thus can be usefully applied to the industrial production of branched-chain amino acids.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Furthermore, those skilled in the art may be able to recognize or identify numerous equivalents to the particular aspects of the present disclosure described herein by using routine experimentation. Moreover, these equivalents are intended to be included in the present disclosure.

As used in the specification and appended claims of the present disclosure, the singular articles ("a", "an", and "the") include plural referents unless the context clearly indicates otherwise. Further, unless the context indicates otherwise, singular terms include their plural forms, and plural terms include their singular forms. As used in the specification and appended claims of the present disclosure, the use of "or" may comprise the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. As another example, the term "about" may refer to a range of -5% to +5% of the given numerical value. However, it is not limited thereto.

As used herein, terms such as "first, second, third,", "i), ii), iii),", or "(a), (b), (c), (d)" are used to distinguish similar elements and do not imply that the elements are executed continuously or in the listed order. For example, when these terms are used in relation to a method, use, or analytical step, the steps may be performed without any time intervals, simultaneously, or with intervals of several seconds, minutes, hours, days, or months.

As used herein, the term "consisting essentially of" may mean that, in the case where the features of the object claimed herein are not substantially affected by the presence of an unspecified component, the unspecified component may be present.

As used herein, the term "consisting of" means that the total ratio of the specific component(s) is 100%. The components or features recited after the term "consisting of" may be essential or mandatory. In some specific embodiments, in addition to the components or features recited after the term "consisting of", other arbitrary or non-essential components may be excluded.

As used herein, the term "comprising" refers to the presence of the features, steps, or components following the term and does not exclude the presence or addition of one or more other features, steps, or components. As used herein, the components or features following the term "comprising" may be essential or mandatory; however, in certain embodiments, other optional or non-essential components or features may also be included.

One aspect of the present disclosure provides a microorganism of the genus *Corynebacterium,* into which a threonine dehydratase derived from *Arabidopsis thaliana* is introduced.

As used herein, the term "threonine dehydratase (*ilvA*)" refers to an enzyme involved in the L-isoleucine biosynthesis pathway. It means an enzyme that produces 2-ketobutyrate from L-threonine, which is an amino acid derived from aspartate (aspartic acid), in the L-isoleucine biosynthetic pathway. L-isoleucine is produced using 2-ketobutyrate and pyruvate produced in the corresponding process (glycolysis) as precursors. The threonine dehydratase of the present disclosure may be used interchangeably with *ilvA* or L-threonine dehydratase. Specifically, the threonine dehydratase of the present disclosure may be a protein exhibiting activity of threonine dehydratase encoded by an *ilvA* gene, but the type thereof is not particularly limited as long as it exhibits activity corresponding to the threonine dehydratase. The threonine dehydratase encoded by an *ilvA* gene is known in the art, and the amino acid and polynucleotide sequences of the threonine dehydratase can be obtained from publicly available databases, examples of which include GenBank of NCBI, but are not limited thereto.

In one example, the threonine dehydratase derived from *Arabidopsis thaliana* of the present disclosure may specifically be a threonine dehydratase derived from the amino acid sequence of SEQ ID NO: 13 or SEQ ID NO: 15, which excludes only a transit peptide from SEQ ID NO: 13, but is not limited thereto.

For example, the threonine dehydratase derived from *Arabidopsis thaliana may* comprise the amino acid sequence of SEQ ID NO: 15 or an amino acid sequence having at least 75% homology or identity thereto, but is not limited thereto as long as it retains threonine dehydratase activity. Specifically, the polypeptide exhibiting threonine dehydratase activity may have or comprise the amino acid sequence of SEQ ID NO: 15 or an amino acid sequence having at least 75%, 80%, 85%, 90%, 95%, 96%, 97%, 98%, or 99% homology or identity to SEQ ID NO: 15, consist of the amino acid sequence, or essentially consist of the amino acid sequence.

With respect to amino acid sequences in the present disclosure, even if it is disclosed as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that a protein having an amino acid sequence in which a portion of the sequence is deleted, modified, substituted, conservatively substituted or added falls within the scope of the present disclosure if it has the same or corresponding activity as the protein consisting of the amino acid sequence of the corresponding sequence number. For example, when exhibiting identical or equivalent activity to the variant protein, it is obvious that a protein with sequence additions, naturally-occurring mutations, or silent mutations or conservative substitutions thereof that do not alter the protein function is not excluded, and that such proteins having sequence additions or mutations fall within the scope of the present disclosure.

For example, the amino acid sequence may include the addition of sequences, naturally occurring mutations, silent mutations, or conservative substitutions that do not alter the function of the variant polypeptide of the present disclosure at the N-terminus and/or C-terminus of the sequence and/or within the amino acid sequence. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamate and aspartate; amino acids with nonpolar side chains (nonpolar amino acids) comprise glycine, alanine, valine, leucine, isoleucine, methionine, phenylalanine, tryptophan, and proline; and amino acids with polar or hydrophilic side chains (polar amino acids) comprise serine, threonine, cysteine, tyrosine, asparagine, and glutamine. In another example, amino acids with electrically charged side chains (electrically charged amino acids) comprise arginine, lysine, histidine, glutamate, and aspartate, and amino acids with electrically uncharged side chains (also referred to as uncharged amino acids or neutral amino acids) comprise glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be categorized as aromatic amino acids. In another example, valine, leucine, and isoleucine may be categorized as branched-chain amino acids (branched amino acids). In another example, 20 types of amino acids may be classified into five groups by size, starting from the group of amino acids with relatively small volumes: glycine, alanine, and serine; cysteine, proline, threonine, aspartate, and asparagine; valine, histidine, glutamate, and glutamine; isoleucine, leucine, methionine, lysine, and arginine; and phenylalanine, tryptophan, and tyrosine. However, the classification of amino acids is not limited thereto. Typically, conservative substitution may have little or no effect on the activity of a polypeptide.

In addition, a base sequence encoding the threonine dehydratase derived from *Arabidopsis thaliana* may be a base sequence encoding a protein that exhibits threonine dehydratase activity and that, in a microorganism of the genus *Corynebacterium,* imparts the activity to enhance branched-chain amino acid production capability.

In one example, the threonine dehydratase derived from *Arabidopsis thaliana* of the present disclosure, specifically a threonine dehydratase derived from the amino acid sequence of SEQ ID NO: 13 or SEQ ID NO: 15, which excludes only the transit peptide from SEQ ID NO: 13, may be encoded by a polynucleotide comprising the base sequence of SEQ ID NOS: 14 and 16, respectively, but is not limited thereto.

In one example, the threonine dehydratase having the amino acid sequence of SEQ ID NO: 15 may be encoded by a polynucleotide having or comprising the sequence of SEQ ID NO: 16 or a base sequence having at least 75%, at least 80%, at least 85%, at least 90%, at least 95%, at least 96%, at least 97%, or at least 98%, and less than 100% homology or identity to the sequence of SEQ ID NO: 16, consisting of the base sequence, or essentially consisting of the base sequence, but is not limited thereto.

In the present disclosure, for example, the gene comprising the base sequence of SEQ ID NO: 16 may be used interchangeably with a polynucleotide comprising the base sequence of SEQ ID NO: 16; a gene or polynucleotide having the base sequence of SEQ ID NO: 16; a gene or polynucleotide consisting of the base sequence of SEQ ID NO: 16; or *ilvA* derived from *Arabidopsis thaliana.*

The polynucleotide of the present disclosure may undergo various modifications in the coding region within the scope that does not change the amino acid sequence of the threonine dehydratase of the present disclosure, taking into consideration codon degeneracy or the codons preferred in an organism in which the threonine dehydratase of the present disclosure is to be expressed. Accordingly, it is apparent that a polynucleotide that can be translated, by codon degeneracy, into a polypeptide consisting of an amino acid sequence of the threonine dehydratase of the present disclosure or a polypeptide having homology or identity thereto may also be comprised. For example, the polynucleotide of the present disclosure may be the sequence of SEQ ID NO: 16, or a degenerate sequence thereof.

In another example, the polynucleotide of the present disclosure may have or comprise a base sequence having at least 75%, at least 80%, at least 85%, at least 90%, at least 95%, at least 96%, at least 97%, or at least 98%, and less than 100% homology or identity to the sequence of SEQ ID NO: 16, or may consist of or essentially consist of a base sequence having at least 75%, at least 80%, at least 85%, at least 90%, at least 95%, at least 96%, at least 97%, or at least 98%, and less than 100% homology or identity to the sequence of SEQ ID NO: 16, but is not limited thereto.

Additionally, the polynucleotide of the present disclosure may include, without limitation, a probe that can be prepared from a known gene sequence, for example, any polynucleotide sequence that hybridizes with a sequence complementary to all or part of the polynucleotide sequence of the present disclosure under stringent conditions to encode the threonine dehydratase of the present disclosure.

As used herein, the term "homology" or "identity" refers to the degree of relevance between two given amino acid sequences or base sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length sequence or to at least about 50%, 60%, 70%, 80%, or 90% or more of the full-length sequence. It is apparent that hybridization also includes hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, and FASTA (Atschul, S. F.et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology, similarity, or identity may be determined by using BLAST of the National Center for Biotechnology Information or ClustalW.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program may be defined as the value obtained by dividing the number of similarly aligned symbols (*i.e.,* nucleotides or amino acids) by the total number of symbols in the shorter of the two sequences. Default parameters for the GAP program may include: (1) a unitary matrix (containing a value of 1 for identities and 0 for non-identities), PAM matrix (see Schwartz and Dayhoff, eds., Atlas Of Protein Sequence And Structure, National Biomedical Research Foundation (1978)), or the weighted comparison matrix (or EDNAFULL (EMBOSS version of NCBI NUC4.4) substitution matrix) of Gribskov et al. (1986) Nucl Acids Res. 14:6745; (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined. Appropriate hybridization conditions are within the skill of the art, and may be determined by a method well known to those skilled in the art (*e.g.,* J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the method for identifying homology, similarity, or identity and the appropriate hybridization conditions are not limited thereto.

As used herein, the term "stringent conditions" refers to conditions that enable specific hybridization between polynucleotides. These conditions are specifically described in the literature (see Sambrook *et al.,* supra, 9.50-9.51, 11.7-11.8). For example, the stringent conditions may include conditions under which polynucleotides having high homology or identity, *i.e.,* polynucleotides having at least 60%, at least 70%, at least 75%, at least 80%, at least 85%, at least 90%, at least 95%, at least 96%, at least 97%, at least 98%, or at least 99% homology or identity hybridize with each other, while polynucleotides having homology or identity lower than the above do not hybridize with each other; or may include ordinary washing conditions of Southern hybridization, *i*.*e*., washing once, specifically twice or three times, at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleotides have complementary sequences, although base mismatches are permissible depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may also comprise an isolated nucleic acid fragment complementary to the entire sequence as well as a base sequence substantially similar thereto.

For example, polynucleotides having homology or identity to the polynucleotide of the present disclosure may be detected using hybridization conditions comprising a hybridization step at a Tₘ value of 55 °C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (*e.g.,* Sambrook *et al.,* supra).

As used herein, the term "microorganism (or strain)" includes both wild-type microorganisms and microorganisms that have undergone genetic modification naturally or artificially. It may be a microorganism in which a specific mechanism is weakened or enhanced due to insertion of a foreign gene or enhancement or inactivation of the activity of an endogenous gene, and may be a microorganism comprising a genetic modification for production of a polypeptide, protein, or product of interest. In the present disclosure, the terms "microorganism" and "strain" have the same meaning and may be used interchangeably without limitation.

For example, the microorganism of the present disclosure may be a microorganism (*e.g.,* recombinant strain) into which a threonine dehydratase derived from *Arabidopsis thaliana* is introduced, but is not limited thereto. The microorganism may be a microorganism having the ability to produce a branched-chain amino acid.

As used herein, the term "microorganism having the ability to produce a branched-chain amino acid" refers to a prokaryotic or eukaryotic microbial strain capable of producing a branched-chain amino acid in a living organism, and may comprise both microorganisms wherein the ability to produce a branched-chain amino acid is imparted to a parent strain with no ability to produce a branched-chain amino acid and microorganisms inherently exhibiting the ability to produce a branched-chain amino acid. The ability to produce a branched-chain amino acid may be imparted or enhanced by strain improvement.

As used herein, the term "branched-chain amino acid" refers to an amino acid with a branched alkyl group in the side chain, and comprises valine, leucine, and isoleucine. Specifically, in the present disclosure, the branched-chain amino acid may be an L-branched-chain amino acid, and the L-branched-chain amino acid may be L-valine, L-leucine, or L-isoleucine, and more specifically L-valine or L-leucine. As used herein, the term "non-modified microorganism" does not exclude strains including mutations that may naturally occur in microorganisms and may refer to a wild-type strain or native strain as-is, or a strain before its trait is modified due to a genetic mutation caused by natural or artificial factors. The "non-modified microorganism" may be used interchangeably with "strain before modification", "microorganism before modification", "non-mutated strain", "non-modified strain", "non-mutant microorganism", "parent strain before mutation", "wild-type microorganism", "reference microorganism", or "control microorganism". As used herein, the non-modified microorganism may refer to a strain into which the threonine dehydratase of the present disclosure has not been introduced or before introduction of the threonine dehydratase of the present disclosure, but is not limited thereto. Additionally, in the present disclosure, the non-modified microorganism may be a microorganism not comprising the amino acid sequence of SEQ ID NO: 13 or 15; or the base sequence of SEQ ID NO: 14 or 16, but is not limited thereto.

For the purposes of the present disclosure, the microorganism of the present disclosure may comprise any microorganism into which a threonine dehydratase derived from *Arabidopsis thaliana* is introduced. Additionally, the microorganism of the present disclosure may comprise any microorganism into which a threonine dehydratase derived from *Arabidopsis thaliana* is introduced, wherein the introduction enables the production of a desired branched-chain amino acid. For example, the microorganism of the present disclosure may be characterized by enhanced ability to produce a branched-chain amino acid resulting from the introduction of a threonine dehydratase derived from *Arabidopsis thaliana,* and may be a genetically modified microorganism or recombinant microorganism, but is not limited thereto. Specifically, the recombinant strain with enhanced ability to produce a branched-chain amino acid may be a microorganism with enhanced ability to produce a branched-chain amino acid compared to a natural wild-type microorganism, a non-modified microorganism having endogenous activity of threonine dehydratase, or a non-modified microorganism with no endogenous activity of threonine dehydratase, but is not limited thereto.

In one example, the microorganism having the ability to produce a branched-chain amino acid refers to a prokaryotic or eukaryotic microbial strain capable of producing a branched-chain amino acid in a living organism, and may comprise both microorganisms inherently exhibiting the ability to produce a branched-chain amino acid and microorganisms wherein the ability to produce a branched-chain amino acid is imparted to a parent strain with no ability to produce a branched-chain amino acid by introduction of the threonine dehydratase derived from *Arabidopsis thaliana* of the present disclosure. The ability to produce a branched-chain amino acid may be imparted or enhanced by strain improvement.

The microorganism of the present disclosure may comprise any microorganism into which a threonine dehydratase derived from *Arabidopsis thaliana* is introduced by various known methods.

As used herein, the term "enhancement" in activity of a polypeptide refers to an increase in the activity of the polypeptide compared to its endogenous activity. The enhancement may be used interchangeably with terms such as activation, up-regulation, overexpression, increase, *etc.*

The "enhancement" may encompass both the exhibition of an activity that was not originally possessed and the exhibition of improved activity compared to the endogenous activity or activity before modification. For example, the "exhibition of an activity that was not originally possessed" may be "introduction of a protein", but is not limited thereto.

As used herein, the term "introduction" of activity refers to a case in which a gene not originally possessed by a microorganism is expressed within the microorganism so that the activity of a specific protein is exhibited, or to an increase, enhancement, or improvement in the activity of a polypeptide compared to its endogenous activity or activity before modification. For example, the activity may be exhibited by introducing a polynucleotide encoding a specific protein into the chromosome of a microorganism or introducing a vector comprising a polynucleotide encoding a specific protein into a microorganism.

The term "endogenous activity" refers to the activity of a specific polypeptide originally possessed by the parent strain before the trait is changed or by an unmodified microorganism when the trait is changed by genetic variation due to natural or artificial factors. This term may be used interchangeably with "activity before modification".

The enhancement in activity of a polypeptide relative to the endogenous activity refers to an improvement in the activity and/or concentration (expression level) of a particular polypeptide naturally possessed by a parent strain before transformation or a non-modified microorganism.

In one example, the enhancement may refer to the emergence of activity of the corresponding protein (polypeptide) that was absent, or the enhancement in activity or concentration of the protein relative to the activity or concentration of a wild-type protein or in a microbial strain before modification, typically by about 1%, about 10%, about 25%, about 50%, about 75%, about 100%, about 150%, about 200%, about 300%, about 400%, or about 500%, up to about 1,000%, or about 2,000% or more, but is not limited thereto.

The enhancement in activity of a polypeptide may be achieved by introducing a foreign polypeptide or enhancing the activity of an endogenous polypeptide. The enhancement in activity of a polypeptide may be confirmed by enhancement in the level of activity or expression of the polypeptide or the amount of product secreted from the polypeptide.

The enhancement in activity of a polypeptide may be achieved by applying various methods well known in the art, and the method is not limited as long as it enhances the activity of a desired polypeptide compared to a microorganism before modification. Specifically, it may be achieved using genetic engineering and/or protein engineering well known to those skilled in the art, which are routine molecular biology techniques, but is not limited thereto (e.g., Sitnicka et al. Functional Analysis of Genes. Advances in Cell Biology. 2010, Vol. 2. 1-16, Sambrook et al. Molecular Cloning 2012, *etc*.)*.*

Specifically, the enhancement in the activity of a polypeptide may be achieved by:
1) increase in the intracellular copy number of a polynucleotide encoding the polypeptide;
2) modification of a gene expression regulatory region on a chromosome encoding the polypeptide (*e.g.,* occurrence of a modification on the expression regulatory region, replacement with a sequence exhibiting stronger activity, or insertion of a sequence exhibiting stronger activity);
3) modification of a base sequence encoding the initiation codon or 5'-UTR region of a gene transcript encoding the polypeptide;
4) modification of the amino acid sequence of the polypeptide so as to enhance the activity of the polypeptide;
5) modification of a polynucleotide sequence encoding the polypeptide such that the activity of the polypeptide is enhanced (*e.g.,* modification of a polynucleotide sequence of the polypeptide gene such that the polynucleotide sequence encodes a polypeptide that has been modified to exhibit enhanced activity of the polypeptide);
6) introduction of a foreign polypeptide exhibiting the activity of the polypeptide, or a foreign polynucleotide encoding the same;
7) codon optimization of a polynucleotide encoding the polypeptide;
8) selection and modification or chemical modification of exposed regions of the polypeptide through analysis of the tertiary structure thereof;
9) regulation of the cellular localization of the polypeptide; or
10) a combination of at least two selected from 1) to 9) above, but is not particularly limited thereto.

More specifically,
The 1) enhancement in the intracellular copy number of a polynucleotide encoding the polypeptide may be achieved by introducing a vector to which the polynucleotide encoding the polypeptide is operably linked and which is capable of replicating and functioning independently of the host, into a host cell. Alternatively, the method may be achieved by introducing one copy, or at least two copies, of a polynucleotide encoding the polypeptide into the chromosome of a host cell. The introduction into the chromosome may be performed by introducing a vector that is capable of inserting the polynucleotide into the chromosome of a host cell, into the host cell, but is not limited thereto. The vector is as described above.

The 2) replacement of the expression regulatory region (expression regulatory sequence) of a gene encoding the polypeptide on the chromosome with a sequence having strong activity may be achieved, for example, by introducing a modification on the sequence through deletion, insertion, non-conservative or conservative substitution, or a combination thereof such that the activity of the expression regulatory region is further enhanced, or by replacing the sequence with a sequence having stronger activity. The expression regulatory region may comprise, but is not particularly limited to, a promoter, an operator sequence, a sequence encoding a ribosome-binding site, a sequence regulating the termination of transcription and translation, and the like. In one example, it may specifically comprise replacing the original promoter with a strong promoter, but is not limited thereto.

Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, but the strong promoter is not limited thereto.

The 3) modification of the base sequence encoding the initiation codon or 5'-UTR of the gene transcript encoding the polypeptide may be, for example, replacing the base sequence with a base sequence encoding another initiation codon that has a higher polypeptide expression rate than the endogenous initiation codon, but is not limited thereto.

The 4) and 5) modification of the amino acid sequence or polynucleotide sequence may be achieved by inducing a mutation in the amino acid sequence of the polypeptide or the polynucleotide sequence encoding the polypeptide through deletion, insertion, non-conservative or conservative substitution, or a combination thereof such that the activity of the polypeptide is enhanced, or by replacing the sequence with an amino acid sequence or polynucleotide sequence modified to have stronger activity or with an amino acid sequence or polynucleotide sequence modified to have enhanced activity, but are not limited thereto. The replacement may specifically be performed by inserting the polynucleotide into the chromosome by homologous recombination, but is not limited thereto. The vector used herein may further comprise a selection marker for confirming chromosomal insertion. The selection marker is as described above.

The 6) introduction of a foreign polynucleotide exhibiting the activity of the polypeptide may be achieved by introducing a foreign polynucleotide encoding a polypeptide that exhibits identical or similar activity to that of the polypeptide, into a host cell. The foreign polynucleotide is not limited by its origin or sequence, as long as it exhibits identical or similar activity to that of the polypeptide. The introduction may be performed by a transformation method known in the art appropriately selected by those skilled in the art, and the expression of the polynucleotide introduced in the host cell enables the production of the polypeptide, thereby enhancing its activity.

The 7) codon-optimization of the polynucleotide encoding the polypeptide may be codon-optimizing the endogenous polynucleotide such that the transcription or translation the same is enhanced within a host cell, or by optimizing a foreign polynucleotide such that the transcription or translation of the same is optimized within the host cell.

The 8) analysis of the tertiary structure of the polypeptide and thereby selecting and modifying exposed sites or chemically modifying the same may be, for example, comparing the sequence information of the polypeptide to be analyzed with a database storing the sequence information of known proteins to identify template protein candidates according to the degree of sequence similarity, confirming the structure based on the information, and thereby selecting and transforming or modifying the exposed sites to be modified or chemically modified.

The 9) regulation of the cellular localization of the polypeptide may be achieved by targeting the polypeptide to a particular organelle or a particular space within the cell. For example, it may be achieved by targeting the polypeptide to the periplasm or cytoplasm through addition or removal of a leader sequence that functions in protein targeting, but is not limited thereto.

Such increase in activity of a polypeptide may be an enhancement of the activity or concentration of the corresponding polypeptide relative to the activity or concentration of the polypeptide expressed in a wild-type strain or a microorganism before modification, or an enhancement of the amount of product produced by the polypeptide, but is not limited thereto.

For example, the recombinant microorganism having the ability to produce a branched-chain amino acid of the present disclosure may comprise any microorganism transformed with a vector, capable of producing a branched-chain amino acid by introducing a foreign gene encoding the threonine dehydratase derived from *Arabidopsis thaliana* of the present disclosure.

The vector of the present disclosure may comprise a DNA construct comprising the base sequence of a polynucleotide encoding a desired polypeptide that is operably linked to an expression regulatory region suitable for expressing the target polypeptide in an appropriate host. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.* may be used; and as a plasmid vector, those based on pDZ, pBR, pUC, pBluescriptll, pGEM, pTZ, pCL, pSK, pSKH, pET, *etc.* may be used. Specifically, pDZ, pDC, pDC24, pACYC177, pACYC184, pCL, pSK, pSKH130, pECCG117, pUC19, pBR322, pMW118, pCC1BAC vector, *etc.* may be used.

In an embodiment, a polynucleotide encoding a desired polypeptide may be inserted into a chromosome through a vector for chromosomal insertion in a cell. The insertion of a polynucleotide into the chromosome may be performed by any method known in the art, such as homologous recombination, but is not limited thereto. A selection marker for confirming the chromosomal insertion may be further comprised. The selection marker is used to screen cells transformed with the vector, *i.e.,* to confirm the insertion of a desired nucleic acid molecule; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a different phenotype, allowing for the selection of transformed cells.

As used herein, the term "transformation" refers to the introduction of a vector comprising a polynucleotide encoding a target polypeptide into a host cell to enable the expression of the polypeptide encoded by the polynucleotide within the host cell. As long as the transformed polynucleotide can be expressed within the host cell, the transformed polynucleotide may be located within the chromosome of the host cell or exist extrachromosomally. Further, the polynucleotide comprises DNA and/or RNA encoding the polypeptide. The polynucleotide may be introduced in any form, provided that it can be introduced into and expressed within a host cell. For example, the polynucleotide may be introduced into a host cell in the form of an expression cassette, which is a gene construct comprising all elements necessary for self-expression. The expression cassette may comprise a promoter operably linked to the polynucleotide, a transcription termination signal, a ribosome binding site, and a translation termination signal. The expression cassette may be in the form of a self-replicating expression vector. Further, the polynucleotide may be introduced into a host cell as-is and operably linked to a sequence necessary for expression in the host cell, but is not limited thereto.

As used herein, the term "operably linked" refers to a configuration in which a regulatory sequence is positioned at an appropriate position to regulate the expression of a coding sequence. Thus, the term "operably linked" comprises attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity. For example, it means that a polynucleotide encoding the desired variant polypeptide of the present disclosure is functionally linked to a promoter sequence that initiates and mediates transcription of the polynucleotide.

As used herein, the term "expression" comprises any step involved in the production of a polypeptide, for example, transcription, post-transcriptional modification, translation, post-translational modification, secretion, and the like, but is not limited thereto.

As used herein, the term "expression vector" refers to a linear or circular nucleic acid molecule comprising a coding sequence and an operably linked regulatory sequence for expression.

As used herein, the term "regulatory sequence" refers to a polynucleotide sequence necessary for the expression of a coding sequence. Each regulatory sequence may be native (derived from the same origin) or foreign (derived from a different gene) to the coding sequence. Examples of the regulatory sequence may comprise a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may comprise a promoter and a sequence for terminating transcription and translation.

As used herein, the term "recombinant" with respect to a cell, or nucleic acid, polypeptide, or vector, means that the cell, polynucleotide, polypeptide, or vector has been modified by the introduction of a heterologous polynucleotide or polypeptide, or the alteration of a native polynucleotide or polypeptide, or that the cell is derived from a cell thus modified. Therefore, for example, recombinant cells may express genes that are not found within the native (non-recombinant) form of the cells, or may express native genes that are otherwise abnormally expressed, under-expressed or not expressed at all.

For example, the microorganism producing a branched-chain amino acid may be a microorganism into which a sequence encoding a protein consisting of the amino acid sequence of SEQ ID NO: 15 or a protein comprising an amino acid sequence having at least 75%, 80%, 85%, 90%, 95%, 96%, 97%, 98%, 99%, 99.5%, 99.7%, or 99.9% or more homology or identity to the amino acid sequence of SEQ ID NO: 15 is introduced.

For example, the microorganism producing a branched-chain amino acid may be a microorganism into which a base sequence capable of encoding a protein comprising an amino acid sequence having at least 75% homology with the amino acid sequence of SEQ ID NO: 15; the base sequence of SEQ ID NO: 16; or a base sequence having at least 75%, at least 80%, at least 85%, at least 90%, at least 95%, at least 96%, at least 97%, or at least 98%, and less than 100% homology or identity to the base sequence of SEQ ID NO: 16 is introduced.

In one example, the microorganism with enhanced ability to produce a branched-chain amino acid of the present disclosure may be a microorganism with enhanced ability to produce a branched-chain amino acid compared to a non-modified microorganism, but is not limited thereto. In one example, the non-modified microorganism, which is the reference strain for comparing the enhancement of L-valine-producing ability, may be *Corynebacterium glutamicum* KCCM11201P (US 8,465,962 B) strain or CJ7V (US 2020-0362374 A1) strain. In another example, the non-modified microorganism, which is the reference strain for comparing the enhancement of L-leucine-producing ability, may be *Corynebacterium glutamicum* CJL8109 (WO 2022/163981 A1) strain, but the non-modified microorganism is not limited thereto.

In one example, the recombinant microorganism having enhanced ability to produce a branched-chain amino acid may exhibit an enhancement of at least about 1%, specifically, at least about 1%, at least about 2.5%, at least about 5%, at least about 6%, at least about 7%, at least about 8%, at least about 9%, at least about 10%, at least about 15%, at least about 20%, at least about 25%, at least about 26%, or at least about 27% (the upper limit is not particularly limited, for example, at most about 200%, at most about 150%, at most about 100%, at most about 50%, at most about 45%, at most about 40%, at most about 35%, or at most about 30%) compared to the ability to produce a branched-chain amino acid of a parent strain before modification or a non-modified microorganism. However, the ability to produce a branched-chain amino acid of the recombinant microorganism is not limited thereto, as long as it exhibits a positive increase compared to the producing ability of a parent strain before modification or a non-modified microorganism. In another example, the microorganism having enhanced ability to produce a branched-chain amino acid may have an enhancement of at least about 1.1-fold, at least about 1.15-fold, at least about 1.20-fold, at least about 1.25-fold, at least about 1.26-fold, or at least about 1.27-fold (the upper limit is not particularly limited, for example, at most about 10-fold, at most about 5-fold, at most about 3-fold, at most about 2-fold, at most about 1.5-fold, at most about 1.4-fold, or at most about 1.3-fold) compared to that of a parent strain before modification or a non-modified microorganism, but is not limited thereto.

In one example, the microorganism may be a microorganism of the genus *Corynebacterium.*

In one example, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the microorganism of the present disclosure may be a microorganism of the genus *Corynebacterium,* and more specifically *Corynebacterium glutamicum,* but is not limited thereto.

The microorganism of the present disclosure may be a microorganism of the genus *Corynebacterium* in which endogenous threonine dehydratase activity is further deleted, but is not limited thereto.

As used herein, the term "weakening" of activity of a polypeptide is a concept encompassing both weakened activity compared to its endogenous activity and absence of activity. The weakening may be used interchangeably with terms such as deficiency, inactivation, deletion, disruption, down-regulation, decrease, reduction, attenuation, repression, and reduction.

For example, the weakening refers to a state in which the protein exhibits activity but is not completely inactivated by deletion. It may mean that the protein activity is weakened relative to a non-modified microorganism, a wild-type strain, or a parent strain, but is not limited thereto.

For example, the weakening may be inactivation or deletion, but is not limited thereto. The inactivation may mean that the protein is not expressed or, even when expressed, no activity or weakened activity is observed compared to a parent strain or a non-modified strain.

The weakening may also include cases where: the activity of a polypeptide is weakened or removed compared to the activity of the polypeptide originally possessed by a microorganism due to mutation of a polynucleotide encoding the polypeptide, *etc.;* the overall activity level and/or concentration (expression level) of the intracellular polypeptide is decreased compared to that of a natural strain due to the inhibition of expression of a gene of a polynucleotide encoding the polypeptide or due to the inhibition of translation into the polypeptide, *etc.;* the polynucleotide is not expressed at all; and/or no polypeptide activity is observed even when the polynucleotide is expressed.

The weakening of activity of a polypeptide compared to the endogenous activity refers to a reduction in the activity of a particular polypeptide naturally possessed by a parent strain before transformation or a non-modified microorganism. The weakening of activity of a polypeptide may be confirmed by weakening of the level of activity or expression of the polypeptide or the amount of product secreted from the polypeptide.

In one example, the weakening may be a case in which the activity of a protein is less than about 100%, about 90% or less, about 80% or less, about 70% or less, about 60% or less, about 50% or less, about 40% or less, about 30% or less, about 20% or less, about 10% or less, about 5% or less, or 0% of the activity of the protein in a parent strain before transformation or a non-modified microorganism, but is not limited thereto.

For example, the inactivation or deletion may mean that the protein is not expressed or, even when expressed, no activity or weakened activity is observed compared to a non-modified microorganism.

The weakening of activity of a polypeptide may be performed by any method known in the art, but the method is not limited thereto, and may be achieved by applying various methods well known in the field (*e.g.,* Nakashima N et al., Bacterial cellular engineering by genome editing and gene silencing. Int J Mol Sci. 2014;15(2):2773-2793, Sambrook et al. Molecular Cloning 2012).

Specifically, the weakening of activity of a polypeptide of the present disclosure may be:
1) deletion of all or part of a gene encoding the polypeptide;
2) modification of an expression control region (or expression control sequence) to decrease the expression of a gene encoding the polypeptide;
3) modification of the amino acid sequence constituting the polypeptide so that the activity of the polypeptide is eliminated or weakened (*e.g.,* deletion/substitution/addition of at least one amino acid in the amino acid sequence);
4) modification of a polynucleotide sequence encoding the polypeptide so that the activity of the polypeptide is eliminated or weakened (*e.g.,* deletion/substitution/addition of at least one nucleic acid in the nucleic acid sequence of the polypeptide gene so that it encodes a polypeptide modified such that the activity thereof is eliminated or it exhibits weakened activity of the polypeptide);
5) modification of a base sequence encoding the initiation codon or 5'-UTR region of a gene encoding the polypeptide;
6) introducing an antisense oligonucleotide (*e.g.,* antisense RNA) that binds complementarily to the gene transcript encoding the polypeptide;
7) addition of a sequence complementary to the Shine-Dalgarno (SD) sequence upstream of the SD sequence of a gene encoding the polypeptide to form a secondary structure that prevents ribosome attachment;
8) reverse transcription engineering (RTE), which adds a promoter to be reversely transcribed on the 3' terminus of the open reading frame (ORF) of a polynucleotide sequence encoding the polypeptide;
9) regulation of the cellular localization of the polypeptide; or
10) a combination of at least two selected from 1) to 9) above, but is not particularly limited thereto.

For example,
The 1) deletion of part or all of a gene encoding the polypeptide may be achieved by deleting all of a polynucleotide encoding a desired endogenous polypeptide within the chromosome or by replacing the polynucleotide with a polynucleotide in which part of the nucleotide sequence is deleted or with a marker gene.

The method of deleting part or all of a polynucleotide may be performed by a method of deleting a polynucleotide by homologous recombination using a chromosomal integration vector in a microorganism, or by inducing mutations using light such as ultraviolet light or a chemical agent and selecting, from the obtained mutants, a strain in which the target gene is deleted, but is not limited thereto. The method of deleting part or all of the gene may include a method based on DNA recombination technology. For example, deletion of all or part of a gene may be achieved by introducing into a microorganism a nucleotide sequence or vector comprising a nucleotide sequence having homology to the desired gene, thereby inducing homologous recombination. The nucleotide sequence or vector to be introduced may comprise a dominant selection marker, but is not limited thereto.

The 2) modification of an expression regulatory region may be, for example, the occurrence of a mutation in the expression regulatory region (or expression regulatory sequence) through deletion, addition, non-conservative or conservative substitution, or a combination thereof, or replacement of the sequence with a sequence having weaker activity. The expression regulatory region may include a promoter, an operator sequence, a sequence encoding a ribosome binding site, and a sequence for regulating the termination of transcription and translation, but is not limited thereto.

In addition, the 3) and 4) modification of the amino acid sequence or polynucleotide sequence may be achieved by inducing a mutation in the amino acid sequence of the polypeptide or the polynucleotide sequence encoding the polypeptide through deletion, insertion, non-conservative or conservative substitution, or a combination thereof such that the activity of the polypeptide is weakened, or by replacing the sequence with an amino acid sequence or polynucleotide sequence modified to have weaker activity or with an amino acid sequence or polynucleotide sequence modified to exhibit no activity, but are not limited thereto. For example, the expression of a gene can be inhibited or weakened by introducing a mutation in a polynucleotide sequence and generating a termination codon, but is not limited thereto.

In addition, the 5) modification of the base sequence encoding the initiation codon or 5'-UTR of the gene encoding the polypeptide may be, for example, substituting the initiation codon with one that has a lower polypeptide expression rate than the endogenous initiation codon, but is not limited thereto.

The 6) introduction of an antisense oligonucleotide (*e.g.,* antisense RNA) that binds complementarily to the gene transcript encoding the polypeptide may be performed in reference to the literature [Weintraub, H. et al., Antisense-RNA as a molecular tool for genetic analysis, Reviews - Trends in Genetics, Vol. 1(1) 1986].

The 7) addition of a sequence complementary to the Shine-Dalgarno (SD) sequence upstream of the SD sequence of a gene encoding the polypeptide to form a secondary structure that prevents ribosome attachment may be inhibiting mRNA translation or reducing the speed thereof.

The 8) reverse transcription engineering (RTE), which adds a promoter to be reversely transcribed on the 3' terminus of the open reading frame (ORF) of a polynucleotide sequence encoding the polypeptide, may be generating an antisense nucleotide complementary to the gene transcript encoding the polypeptide to weaken the activity.

The 9) regulation of the cellular localization of the polypeptide may be achieved by targeting the polypeptide to a particular organelle or a particular space within the cell. For example, it may be achieved by targeting the polypeptide to the periplasm or cytoplasm through addition or removal of a leader sequence that functions in protein targeting, but is not limited thereto.

As a microorganism according to any one of the foregoing specific embodiments, the microorganism of the present disclosure may be a microorganism of the genus *Corynebacterium* into which a threonine dehydratase derived from *Arabidopsis thaliana* is introduced and in which endogenous threonine dehydratase activity is deleted, but is not limited thereto.

The endogenous threonine dehydratase may be derived from a microorganism of the genus *Corynebacterium,* and specifically be a threonine dehydratase derived from *Corynebacterium glutamicum.* The amino acid and polynucleotide sequences of a threonine dehydratase derived from *Corynebacterium glutamicum* can be obtained from publicly available databases, examples of which include GenBank of NCBI, but are not limited thereto.

In one example, the threonine dehydratase derived from *Corynebacterium glutamicum* may comprise the amino acid sequence of SEQ ID NO: 41 or an amino acid sequence having at least 60% homology or identity thereto, but is not limited thereto as long as it retains the activity of threonine dehydratase derived from *Corynebacterium glutamicum.* Specifically, the polypeptide exhibiting activity of threonine dehydratase derived from *Corynebacterium glutamicum* may have, comprise, consist of, or essentially consist of the amino acid sequence of SEQ ID NO: 41 or an amino acid sequence having at least 60%, 70%, 80%, 85%, 90%, 95%, 96%, 97%, 98%, or 99% homology or identity to SEQ ID NO: 41. In one example, the threonine dehydratase derived from *Corynebacterium glutamicum* may refer to a protein inherently present in *Corynebacterium glutamicum,* but is not limited thereto. Specifically, the threonine dehydratase derived from *Corynebacterium glutamicum* may be a threonine dehydratase derived from *Corynebacterium glutamicum* consisting of the amino acid sequence of SEQ ID NO: 41, which is inherently present in *Corynebacterium glutamicum,* but is not limited thereto.

In addition, the base sequence encoding the threonine dehydratase derived from *Corynebacterium glutamicum* may be a base sequence encoding a protein exhibiting the activity of a threonine dehydratase derived from *Corynebacterium glutamicum.*

For example, the threonine dehydratase derived from *Corynebacterium glutamicum* having the amino acid sequence of SEQ ID NO: 41 may be encoded by a polynucleotide having, comprising, consisting of, or essentially consisting of the sequence of SEQ ID NO: 42 or 43 or a base sequence having at least 60%, at least 70%, at least 75%, at least 80%, at least 85%, at least 90%, at least 95%, at least 96%, at least 97%, or at least 98%, and less than 100% homology or identity to the sequence of SEQ ID NO: 42 or 43, but is not limited thereto. The base sequence of SEQ ID NO: 42 or SEQ ID NO: 43 can be obtained from a known database, examples of which include GenBank of NCBI, but is not limited thereto.

Meanwhile, the microorganism of the genus *Corynebacterium* having the ability to produce a branched-chain amino acid of the present disclosure comprises any of a natural wild-type microorganism, a microorganism of the genus *Corynebacterium* with improved ability to produce a branched-chain amino acid by enhancing or weakening the activity of a gene related to the branched chain amino acid production mechanism, or a microorganism of the genus *Corynebacterium* with ability to produce a branched-chain amino acid by introducing or enhancing the activity of an exogenous gene.

Another aspect of the present disclosure provides a method for producing a branched-chain amino acid, comprising culturing the microorganism of the genus *Corynebacterium* of the present disclosure, into which a threonine dehydratase derived from *Arabidopsis thaliana* is introduced, in a medium.

Still another aspect of the present disclosure provides a method for enhancing the production of a branched-chain amino acid, comprising culturing the microorganism of the genus *Corynebacterium* of the present disclosure, into which a threonine dehydratase derived from *Arabidopsis thaliana* is introduced, in a medium.

As used herein, the term "culturing" refers to growing the strain of the present disclosure under properly controlled environmental conditions. The culturing process of the present disclosure may be performed in a suitable medium known in the art under suitable culturing conditions known in the art. Such a culturing process may be easily adjusted and used by those skilled in the art according to the selected strain. Specifically, the culturing may be batch culturing, continuous culturing, and fed-batch culturing, but is not limited thereto.

As used herein, the term "medium" refers to a mixed substance containing nutrients required to culture the microorganism of the present disclosure as a main component, and the medium supplies nutrients, growth factors, *etc.,* including water, which are indispensable for survival and development. Specifically, any medium and culture conditions may be used for culturing the strain of the present disclosure without particular limitation, as long as the medium is used for the common culture of microorganisms. The microorganism of the present disclosure may be cultured in a common medium containing suitable carbon sources, nitrogen sources, phosphorus sources, inorganic compounds, amino acids, and/or vitamins, *etc.,* while controlling the temperature, pH, *etc.* under aerobic conditions. For example, a culture medium for a microorganism of the genus *Corynebacterium* can be found in the document ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.,* molasses converted into reducing sugars) may be used, and other various carbon sources in appropriate amounts may be used without limitation. These carbon sources may be used alone or in combination of at least two, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of at least two, but are not limited thereto.

As the phosphorus sources, monobasic potassium phosphate, dibasic potassium phosphate, or the corresponding sodium-containing salts may be used. As for inorganic compounds, sodium chloride, calcium chloride, iron chloride, magnesium sulfate, iron sulfate, manganese sulfate, calcium carbonate, *etc.,* may be used, and in addition, amino acids, vitamins, and/or suitable precursors may be comprised. These components or precursors may be added to the medium in a batchwise or continuous manner. However, the medium is not limited thereto.

During the culturing of the microorganism of the present disclosure, compounds such as ammonium hydroxide, potassium hydroxide, ammonia, phosphoric acid, and sulfuric acid may be added to the medium in a suitable manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 27°C to 37°C, specifically 30°C to 33°C, and the culturing may be performed for about 20 to 120 hours, but the culturing conditions are not limited thereto.

As used herein, the term "culture" refers to a culture solution, a concentrated culture solution, a dried product of the culture solution, a culture filtrate, a concentrated culture filtrate, or a dried product of the culture filtrate obtained by culturing a specific microorganism in a culture medium. The culture solution refers to a solution comprising a specific microorganism, whereas the culture filtrate refers to a solution that substantially does not contain the specific microorganism (wherein "substantially" means that the specific microorganism separated by filtration or the like is excluded, but does not mean that the microorganism is completely absent from the filtrate). The culture is not limited in its form, and, in one example, may be in the form of a liquid, an emulsion, or a solid. Specifically, for the purpose of the present disclosure, the culture may comprise a branched-chain amino acid.

As used herein, the term "fermentation" refers to a process in which a microorganism decomposes organic matter using enzymes possessed by the microorganism, excluding putrefactive reactions. Fermentation reactions and putrefactive reactions proceed using similar processes. However, upon decomposition, fermentation produces useful substances, whereas putrefaction produces bad odors or harmful substances.

In the present disclosure, the method for obtaining a fermentation product from a strain is not particularly limited and can be obtained according to a method conventionally used in the relevant or similar technical field.

As used herein, the term "fermentation product" includes all types of materials comprising a fermentation product produced by the strain, including not only the fermented material itself, but also a culture medium of a strain in which the strain and a culture coexist; a fermentation product obtained by filtering the strain from the culture medium; a fermentation product obtained by sterilizing the strain in the culture medium and then filtering the same; an extract obtained by extracting the fermentation product or a culture medium comprising the same; a diluted solution obtained by diluting the fermentation product or the extract thereof; a concentrate; a dried product obtained by drying the fermentation product or the extract thereof; and a lysate obtained by collecting and disrupting cells of the strain.

In the method of the present disclosure, culturing of the microorganism may be performed using any culturing conditions and culturing methods known in the art. Such a culturing process may be readily adjusted and used by those skilled in the art according to the selected strain.

A branched-chain amino acid produced by the culturing of the present disclosure may be secreted into the medium or remain in cells.

In one specific embodiment, the method for producing a branched-chain amino acid of the present disclosure may further comprise preparing the microorganism of the present disclosure, preparing a medium for culturing the microorganism, or a combination thereof (in any order), for example, prior to culturing.

The method for producing a branched-chain amino acid of the present disclosure may further comprise recovering a desired substance, specifically a branched-chain amino acid, from the cultured microorganism, a culture of the microorganism, a fermentation product of the microorganism, or the medium after cultivation. The recovering step may be further included after the culturing.

The recovery may be collecting the desired branched-chain amino acid using a suitable method known in the art depending on the method for culturing the microorganism of the present disclosure, such as batch, continuous, or fed-batch culturing. For example, the recovery may involve centrifugation, filtration, treatment with a crystallized protein precipitating agent (salting-out method), extraction, sonication, ultrafiltration, dialysis, various kinds of chromatography such as molecular sieve chromatography (gel filtration), adsorption chromatography, ion exchange chromatography, and affinity chromatography, HPLC or a combination thereof. A desired substance, specifically a branched-chain amino acid, can be recovered from the medium or microorganism using a suitable method known in the art.

Additionally, the method for producing a branched-chain amino acid of the present disclosure may further comprise purification. The purification may be performed using a suitable method known in the art. In one example, when the method for producing a branched-chain amino acid of the present disclosure includes both the recovery and the purification, these steps may be performed continuously or discontinuously in any order, simultaneously, or as one integrated step, but are not limited thereto.

In the method of the present disclosure, threonine dehydratase, introduction, branched-chain amino acid, and the like are as described in other aspects above.

Still another aspect of the present disclosure provides a composition for producing a branched-chain amino acid, comprising: the microorganism of the genus *Corynebacterium* of the present disclosure, into which a threonine dehydratase derived from *Arabidopsis thaliana* is introduced, a culture of the microorganism, a fermentation product of the microorganism, or a combination of at least two thereof.

The composition of the present disclosure may further comprise a suitable excipient commonly used in compositions for producing a branched-chain amino acid. Examples of such excipients include preservatives, wetting agents, dispersing agents, suspending agents, buffering agents, stabilizers, or isotonic agents, but the excipient is not limited thereto.

In one specific embodiment, the composition of the present disclosure may comprise each component in a microbially effective amount or in an amount that can be suitably present in a composition for production.

In the composition of the present disclosure, threonine dehydratase, introduction, branched-chain amino acid, and the like are as described in other aspects above.

Still another aspect of the present disclosure provides a use of the microorganism of the genus *Corynebacterium* of the present disclosure, into which a threonine dehydratase derived from *Arabidopsis thaliana* is introduced, for producing a branched-chain amino acid.

In the use of the present disclosure, threonine dehydratase, introduction, branched-chain amino acid, and the like are as described in other aspects above.

### [Mode for Carrying Out the Invention]

The present disclosure will be described in detail by way of Examples. However, these Examples are given for illustrative purposes only, and the scope of the present disclosure is not intended to be limited by these Examples. Meanwhile, technical descriptions absent in the present disclosure may be sufficiently understood and readily practiced by those skilled in the art of the present disclosure or related art.

### Example 1: Construction of L-valine-producing strains into which a foreign threonine dehydratase was introduced

A foreign threonine dehydratase gene was inserted into the chromosome of *Corynebacterium glutamicum* KCCM11201P (US 8,465,962 B), a valine-producing strain.

### Example 1-1: Construction of L-valine-producing strain in which Endogenous ilvA(Cgl) is deleted

To construct a vector for deletion of a gene (SEQ ID NO: 42) encoding an autologous threonine dehydratase (SEQ ID NO: 41) of *Corynebacterium glutamicum,* PCR was performed using the chromosome of KCCM11201P as a template. The PCR conditions were as follows: an initial denaturation at 95°C for 10 minutes; 28 cycles of denaturation at 95°C for 30 seconds, annealing at 55°C for 40 seconds, and extension at 72°C for 40 seconds; followed by a final extension at 72°C for 5 minutes. As a result, a 643 bp DNA fragment upstream of the autologous *ilvA* 5' region was obtained using primers of SEQ ID NOS: 1 and 2, and a 777 bp DNA fragment downstream of the autologous *ilvA* 3' region was obtained using primers of SEQ ID NOS: 3 and 4.

The two amplified DNA fragments and a pDC24 vector treated with BamHI and Xbal (New England Biolabs, Beverly, MA) were cloned using the In-Fusion Cloning Kit (TaKaRa) in accordance with the provided manual, thereby constructing a *Corynebacterium glutamicum* autologous *ilvA* deletion vector pDC24_ΔilvA.

**[Table 1]**

| Primer | Base sequence (5' → 3') |
|---|---|
| SEQ ID NO: 1 | gctcggtacccggggatccGGTGATTGCGAGCAATCCC |
| SEQ ID NO: 2 | TATGTGGTTGACTAGTGTAATCCTCTCCTAGAATT |
| SEQ ID NO: 3 | CACTAGTCAACCACATAGCTGAAGGCCACCTCAAT |
| SEQ ID NO: 4 | gcctgcaggtcgactctagaTGAACACACCGGAAACTACTCC |
| SEQ ID NO: 5 | GCATTGTTCTACAAAGAGCTCGTT |
| SEQ ID NO: 6 | GAGAACTGGCTATCGATGGC |

The constructed pDC24_ΔilvA was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6 to confirm deletion of the autologous *ilvA.* The strain in which the ORF of the *ilvA* gene was deleted from the chromosome of the parent strain KCCM11201P was named *Corynebacterium glutamicum* KCCM11201P_ΔilvA.

### Example 1-2: Construction of strain into which Arabidopsis thaliana-derived threonine dehydratase (ilvA(Ath)) was introduced

In order to insert a foreign *ilvA* encoding a threonine dehydratase protein, the autologous *ilvA* gene of *Corynebacterium glutamicum* was used as an insertion site. To this end, PCR was performed using the chromosome of KCCM11201P as a template. The PCR conditions were as follows: an initial denaturation at 95°C for 10 minutes; 28 cycles of denaturation at 95°C for 30 seconds, annealing at 55°C for 30 seconds, and extension at 72°C for 50 seconds; followed by a final extension at 72°C for 5 minutes. As a result, a 638 bp DNA fragment upstream of the autologous *ilvA* 5' region of *Corynebacterium glutamicum* (hereinafter referred to as "Fragment A") was obtained using primers of SEQ ID NOS: 7 and 8, and a 776 bp DNA fragment downstream of the autologous *ilvA* 3' region of *Corynebacterium glutamicum* (hereinafter referred to as "Fragment B") was obtained using primers of SEQ ID NOS: 9 and 10.

**[Table 2]**

| Primer | Base sequence (5' → 3') |
|---|---|
| SEQ ID NO: 7 | gctcggtacccggggatccGGTGATTGCGAGCAATCCC |
| SEQ ID NO: 8 | GGTTGACTAGTGTAATCCTCTCCTAGAATT |
| SEQ ID NO: 9 | ACATAGCTGAAGGCCACCTCAATCGAGGTGGC |
| SEQ ID NO: 10 | gcctgcaggtcgactctagaTGAACACACCGGAAACTACTCC |
| SEQ ID NO: 11 | GGATTACACTAGTCAACCatgTCCATGACCCCGCCTCC |
| SEQ ID NO: 12 | AGGTGGCCTTCAGCTATGTTTAGTGCATGAGTAGTTTAA |

Meanwhile, in order to obtain a gene fragment having threonine dehydratase activity, a 1779 bp *Arabidopsis thaliana-derived ilvA* gene (SEQ ID NO: 14) was synthesized (Bionics) by performing codon optimization to be suitable for the codon usage of *Corynebacterium glutamicum* based on the *Arabidopsis thaliana-derived ilvA* amino acid sequence (SEQ ID NO: 13) disclosed in the NCBI database. Subsequently, using the synthesized *Arabidopsis thaliana-derived ilvA* gene as a template, PCR was performed with primers of SEQ ID NOS: 11 and 12 to obtain a 1681 bp DNA fragment (hereinafter referred to as "Fragment C") comprising ilvA(Ath, Δ1-45, T46M), which is a gene (SEQ ID NO: 16) encoding an amino acid sequence (SEQ ID NO: 15) excluding a transit peptide from the amino acid sequence of ilvA(Ath). The PCR conditions were as follows: an initial denaturation at 95°C for 10 minutes; 28 cycles of denaturation at 95°C for 30 seconds, annealing at 55°C for 30 seconds, and extension at 72°C for 1 minute 30 seconds; followed by a final extension at 72°C for 5 minutes.

The previously obtained fragments A, B, and C, and a pDC24 vector treated with BamHI and Xbal (New England Biolabs, Beverly, MA) were cloned using the In-Fusion Cloning Kit (TaKaRa) in accordance with the provided manual, thereby constructing a vector, namely pDC24_ΔilvA::ilvA(Ath), capable of inserting the ilvA(Ath, Δ1-45, T46M) gene into the autologous *ilvA* position of *Corynebacterium glutamicum.*

The constructed pDC24_ΔilvA::ilvA(Ath) was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6, followed by sequence analysis, to confirm whether the autologous *ilvA* ORF was deleted and the *Arabidopsis thaliana-*derived *ilvA* was introduced at the corresponding position. A strain in which the autologous *ilvA* ORF of the parent strain KCCM11201P was replaced with the *Arabidopsis thaliana-derived ilvA* (Ath, Δ1-45, T46M) gene on the chromosome was named *Corynebacterium glutamicum* KCCM11201P_ΔilvA::ilvA(Ath).

### Example 1-3: Construction of strain into which Escherichia coli-derived threonine dehydratase(ilvA(Eco)) was introduced

In order to confirm the L-valine-producing ability of a strain into which a foreign threonine dehydratase other than an *Arabidopsis thaliana-derived* threonine dehydratase was introduced, an L-valine-producing strain into which an *Escherichia* coli-derived ilvA(Eco) gene was introduced was constructed. As in Example 1-2 above, to use the autologous *ilvA* gene of *Corynebacterium glutamicum* as an insertion site, a 638 bp DNA fragment A upstream of the autologous *ilvA* 5' region was obtained using primers of SEQ ID NOS: 7 and 8, and a 776 bp DNA fragment B downstream of the autologous *ilvA* 3' region was obtained using primers of SEQ ID NOS: 9 and 10.

**[Table 3]**

| Primer | Base sequence (5' → 3') |
|---|---|
| SEQ ID NO: 17 | TTACACTAGTCAACCATGGCTGACTCGCAACCCC |
| SEQ ID NO: 18 | GGTGGCCTTCAGCTATGTCTAACCCGCCAAAAAGAACCTG AAC |

Meanwhile, in order to obtain a gene (SEQ ID NO: 20) fragment encoding an *Escherichia* coli-derived threonine dehydratase (SEQ ID NO: 19), a 1578 bp DNA fragment (hereinafter referred to as "Fragment D") comprising ilvA(Eco) was obtained by performing PCR using chromosomal DNA of *Escherichia coli* MG1655 as a template with primers of SEQ ID NOS: 17 and 18. The PCR conditions were as follows: an initial denaturation at 95°C for 10 minutes; 28 cycles of denaturation at 95°C for 30 seconds, annealing at 55°C for 30 seconds, and extension at 72°C for 1 minute 30 seconds; followed by a final extension at 72°C for 5 minutes.

The previously obtained fragments A, B, and D, and a pDC24 vector treated with BamHI and Xbal (New England Biolabs, Beverly, MA) were cloned using the In-Fusion Cloning Kit (TaKaRa) in accordance with the provided manual, thereby constructing a vector, namely pDC24_ΔilvA::ilvA(Eco), capable of inserting the ilvA(Eco) gene into the autologous *ilvA* position of *Corynebacterium glutamicum.*

The constructed pDC24_ΔilvA::ilvA(Eco) was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6, followed by sequence analysis, to confirm whether the autologous *ilvA* ORF was deleted and the *Escherichia* coli-derived *ilvA* was introduced at the corresponding position. A strain in which the autologous *ilvA* ORF of the parent strain KCCM11201P was replaced with the *Escherichia* coli-derived ilvA(Eco) on the chromosome was named *Corynebacterium glutamicum* KCCM11201P_ΔilvA::ilvA(Eco).

### Example 1-4: Construction of strain into which Solanum lycopersicum-derived threonine dehydratase(ilvA(Sly)) was introduced

In order to confirm the L-valine-producing ability of a strain into which a foreign threonine dehydratase other than an *Arabidopsis thaliana-derived* threonine dehydratase was introduced, an L-valine-producing strain into which a *Solanum lycopersicum*-derived ilvA(Sly) gene was introduced was constructed. As in Example 1-2 above, to use the autologous *ilvA* gene of *Corynebacterium glutamicum* as an insertion site, a 638 bp DNA fragment A upstream of the autologous *ilvA* 5' region was obtained using primers of SEQ ID NOS: 7 and 8, and a 776 bp DNA fragment B downstream of the autologous *ilvA* 3' region was obtained using primers of SEQ ID NOS: 9 and 10.

**[Table 4]**

| Primer | Base sequence (5' → 3') |
|---|---|
| SEQ ID NO: 21 | GATTACACTAGTCAACCATgCTGTCCTCCCCAGCAACC |
| SEQ ID NO: 22 | GTGGCCTTCAGCTATGTTCAGTGCATGATCAGCTGGTATG |

Meanwhile, in order to obtain a gene fragment having threonine dehydratase activity, a 1821 bp *Solanum lycopersicum*-derived *ilvA* gene (SEQ ID NO: 24) was synthesized (Bionics) by performing codon optimization to be suitable for the codon usage of *Corynebacterium glutamicum* based on the *Solanum lycopersicum*-derived *ilvA* amino acid sequence (SEQ ID NO: 23) disclosed in the NCBI database. Subsequently, using the synthesized *Solanum lycopersicum*-derived *ilvA* gene as a template, PCR was performed with primers of SEQ ID NOS: 21 and 22 to obtain a 1696 bp DNA fragment (hereinafter referred to as "Fragment E") comprising ilvA(Sly, Δ1-53, I54M), which is a gene (SEQ ID NO: 26) encoding an amino acid sequence (SEQ ID NO: 25) excluding a transit peptide from the amino acid sequence of ilvA(Sly). The PCR conditions were as follows: an initial denaturation at 95°C for 10 minutes; 28 cycles of denaturation at 95°C for 30 seconds, annealing at 55°C for 30 seconds, and extension at 72°C for 1 minute 30 seconds; followed by a final extension at 72°C for 5 minutes.

The previously obtained fragments A, B, and E, and a pDC24 vector treated with BamHI and Xbal (New England Biolabs, Beverly, MA) were cloned using the In-Fusion Cloning Kit (TaKaRa) in accordance with the provided manual, thereby constructing a vector, namely pDC24_ΔilvA::ilvA(Sly), capable of inserting the ilvA(Sly, Δ1-53, I54M) gene into the autologous *ilvA* position of *Corynebacterium glutamicum.*

The constructed pDC24_ΔilvA::ilvA(Sly) was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6, followed by sequence analysis, to confirm whether the autologous *ilvA* ORF was deleted and the *Solanum lycopersicum-*derived *ilvA* was introduced at the corresponding position. A strain in which the autologous *ilvA* ORF of the parent strain KCCM11201P was replaced with the Solanum lycopersicum-derived ilvA(Sly, Δ1-53, I54M) on the chromosome was named *Corynebacterium glutamicum* KCCM11201P_ΔilvA::ilvA(Sly).

### Example 1-5: Construction of strain into which Spinacia oleracea-derived threonine dehydratase(ilvA(Sol)) was introduced

In order to confirm the L-valine-producing ability of a strain into which a foreign threonine dehydratase other than an *Arabidopsis thaliana-derived* threonine dehydratase was introduced, an L-valine-producing strain into which a *Spinacia* oleracea-derived ilvA(Sol) gene was introduced was constructed. As in Example 1-2 above, to use the autologous *ilvA* gene of *Corynebacterium glutamicum* as an insertion site, a 638 bp DNA fragment A upstream of the autologous *ilvA* 5' region was obtained using primers of SEQ ID NOS: 7 and 8, and a 776 bp DNA fragment B downstream of the autologous *ilvA 3'* region was obtained using primers of SEQ ID NOS: 9 and 10.

**[Table 5]**

| Primer | Base sequence (5' → 3') |
|---|---|
| SEQ ID NO: 27 | ATTACACTAGTCAACCaTGTTCTCCCCAGAAGAGACC |
| SEQ ID NO: 28 | GGTGGCCTTCAGCTATGTTCAGTGCATCAGGACTTCGTAG |

Meanwhile, in order to obtain a gene fragment having threonine dehydratase activity, a 1842 bp *Spinacia* oleracea-derived *ilvA* gene (SEQ ID NO: 30) was synthesized (Bionics) by performing codon optimization to be suitable for the codon usage of *Corynebacterium glutamicum* based on the *Spinacia* oleracea-derived *ilvA* amino acid sequence (SEQ ID NO: 29) disclosed in the NCBI database. Subsequently, using the synthesized *Spinacia* oleracea-derived *ilvA* gene as a template, PCR was performed with primers of SEQ ID NOS: 27 and 28 to obtain a 1714 bp DNA fragment (hereinafter referred to as "Fragment F") comprising ilvA(Sol, Δ1-54, V55M), which is a gene (SEQ ID NO: 32) encoding an amino acid sequence (SEQ ID NO: 31) excluding a transit peptide from the amino acid sequence of ilvA(Sol). The PCR conditions were as follows: an initial denaturation at 95°C for 10 minutes; 28 cycles of denaturation at 95°C for 30 seconds, annealing at 55°C for 30 seconds, and extension at 72°C for 1 minute 30 seconds; followed by a final extension at 72°C for 5 minutes.

The previously obtained fragments A, B, and F, and a pDC24 vector treated with BamHI and Xbal (New England Biolabs, Beverly, MA) were cloned using the In-Fusion Cloning Kit (TaKaRa) in accordance with the provided manual, thereby constructing a vector, namely pDC24_ΔilvA::ilvA(Sol), capable of inserting the ilvA(Sol, Δ1-54, V55M) gene into the autologous *ilvA* position of *Corynebacterium glutamicum.*

The constructed pDC24_ΔilvA::ilvA(Sol) was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6, followed by sequence analysis, to confirm whether the autologous *ilvA* ORF was deleted and the *Spinacia oleracea-*derived *ilvA* was introduced at the corresponding position. A strain in which the autologous *ilvA* ORF of the parent strain KCCM11201P was replaced with the *Spinacia* oleracea-derived ilvA(Sol, Δ1-54, V55M) on the chromosome was named *Corynebacterium glutamicum* KCCM11201P_ΔilvA::ilvA(Sol) .

### Example 1-6: Construction of strain into which Brachypodium distachyon-derived threonine dehydratase(ilvA(Bdi)) was introduced

In order to confirm the L-valine-producing ability of a strain into which a foreign threonine dehydratase other than an *Arabidopsis thaliana-derived* threonine dehydratase was introduced, an L-valine-producing strain into which a *Brachypodium distachyon-*derived ilvA(Bdi) gene was introduced was constructed. As in Example 1-2 above, to use the autologous *ilvA* gene of *Corynebacterium glutamicum* as an insertion site, a 638 bp DNA fragment A upstream of the autologous *ilvA* 5' region was obtained using primers of SEQ ID NOS: 7 and 8, and a 776 bp DNA fragment B downstream of the autologous *ilvA* 3' region was obtained using primers of SEQ ID NOS: 9 and 10.

**[Table 6]**

| Primer | Base sequence (5' → 3') |
|---|---|
| SEQ ID NO: 33 | GATTACACTAGTCAACCATGGAAGTGTTCTCCTTCAAGCTG |
| SEQ ID NO: 34 | GTGGCCTTCAGCTATGTTCATGCCTTTGGGTTGCGCAG |

Meanwhile, in order to obtain a gene fragment having threonine dehydratase activity, a 1392 bp ilvA(Bdi) gene (SEQ ID NO: 36) was synthesized (Bionics) by performing codon optimization to be suitable for the codon usage of *Corynebacterium glutamicum* based on the *Brachypodium distachyon*-derived *ilvA* amino acid sequence (SEQ ID NO: 35) disclosed in the NCBI database. Using the synthesized ilvA(Bdi) gene as a template, PCR was performed with primers of SEQ ID NOS: 33 and 34 to obtain a 1426 bp DNA fragment (hereinafter referred to as "Fragment G") comprising ilvA(Bdi). The PCR conditions were as follows: an initial denaturation at 95°C for 10 minutes; 28 cycles of denaturation at 95°C for 30 seconds, annealing at 55°C for 30 seconds, and extension at 72°C for 1 minute 20 seconds; followed by a final extension at 72°C for 5 minutes.

The previously obtained fragments A, B, and G, and a pDC24 vector treated with BamHI and Xbal (New England Biolabs, Beverly, MA) were cloned using the In-Fusion Cloning Kit (TaKaRa) in accordance with the provided manual, thereby constructing a vector, namely pDC24_ΔilvA::ilvA(Bdi), capable of inserting the ilvA(Bdi) gene into the autologous *ilvA* position of *Corynebacterium glutamicum.*

The constructed pDC24_ΔilvA::ilvA(Bdi) was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 5 and 6, followed by sequence analysis, to confirm whether the autologous *ilvA* ORF was deleted and the *B. distachyon*-derived *ilvA* was introduced at the corresponding position. A strain in which the autologous *ilvA* ORF of the parent strain KCCM11201P was replaced with the B. *distachyon*-derived ilvA(Bdi) on the chromosome was named *Corynebacterium glutamicum* KCCM11201P_ΔilvA::ilvA(Bdi).

### Example 2: Evaluation of L-valine-producing ability of strains into which a foreign threonine dehydratase was introduced

A flask evaluation was performed to compare the valine-producing ability of *Corynebacterium glutamicum* KCCM11201P and six strains constructed in Examples 1-1 to 1-6, namely KCCM11201P_ΔilvA, KCCM11201P ΔilvA::ilvA(Ath), KCCM11201P_ΔilvA::ilvA(Eco), KCCM11201P_ΔilvA::ilvA(Sly), KCCM11201P_ΔilvA::ilvA(Sol), and KCCM11201P_ΔilvA::ilvA(Bdi), all of which are valine producing strains. Each strain was serially subcultured in a nutrient medium, inoculated into a 250 mL corner-baffle flask containing 25 mL of a production medium, and shake-cultured at 30°C and 200 rpm for 72 hours. The valine-producing ability of each strain was measured and is shown in Table 7 below.
[Nutrient Medium (pH 7.2)]
   Glucose 10 g, Meat juice 5 g, Polypeptone 10 g, Sodium chloride 2.5 g, Yeast extract 5 g, Agar 20 g, and Urea 2 g (based on 1 L of distilled water)
[Production Medium (pH 7.0)]
   Glucose 100 g, Ammonium sulfate 40 g, Soy protein 2.5 g, Corn steep solids 5 g, Urea 3 g, Potassium phosphate dibasic 1 g, Magnesium sulfate heptahydrate 0.5 g, Biotin 100 µg, Thiamine-HCl 1 mg, Calcium pantothenate 2 mg, Nicotinamide 3 mg, and Calcium carbonate 30 g (based on 1 L of distilled water)

**[Table 7]**

| Strain | Valine-producing ability |
|---|---|
| | g/L/h |
| KCCM11201P | 0.035 |
| KCCM11201P_Δilva | 0.012 |
| KCCM11201P_ΔilvA::ilvA(Ath) | 0.040 |
| KCCM11201P_ΔilvA::ilvA(Eco) | 0.029 |
| KCCM11201P_ΔilvA::ilvA(Sol) | 0.021 |
| KCCM11201P_ΔilvA::ilvA(Sly) | 0.019 |
| KCCM11201P_ΔilvA::ilvA(Bdi) | 0.015 |

As shown in Table 7 above, KCCM11201P_ΔilvA::ilvA(Ath) exhibited the highest valine-producing ability among the evaluated strains, with a value of 0.040 g/L/h. Specifically, the KCCM11201P_Δ*ilvA* strain in which *ilvA* was deleted showed a very low valine-producing ability compared to the KCCM11201P strain in which *ilvA* was not deleted, whereas KCCM11201P_ΔilvA::ilvA(Ath) exhibited a valine-producing ability corresponding to 114% of that of KCCM11201P. In addition, it was confirmed that introduction of the *Arabidopsis thaliana-derived ilvA* improved the valine-producing ability to a level of 333% relative to the KCCM11201P_ΔilvA strain in which *ilvA* was deleted.

### Example 3: Construction of Corynebacterium glutamicum CJ7V strain into which ilvA(Ath) is introduced and evaluation of L-valine-producing ability

In order to confirm whether introduction of ilvA(Ath) can increase L-valine-producing ability also in other *Corynebacterium glutamicum* strains producing L-valine, a single mutation [ilvN(A42V); Biotechnology and Bioprocess Engineering, June 2014, Volume 19, Issue 3, pp. 456-467] was introduced into the wild-type *Corynebacterium glutamicum* ATCC14067 to construct a strain CJ7V with improved L-valine-producing ability (US 2020-0362374 A1).

Specifically, genomic DNA of the wild-type *Corynebacterium glutamicum* ATCC14067 strain was extracted using a G-spin Total DNA Extraction Mini Kit (Intron, Cat. No. 17045) according to the protocol provided with the kit. PCR was performed using the genomic DNA as a template. In order to construct a vector for introducing the A42V mutation into the *ilvN* gene, gene fragments (H and I) were respectively obtained using a primer pair of SEQ ID NOS: 37 and 38 and a primer pair of SEQ ID NOS: 39 and 40. The PCR conditions were as follows: an initial denaturation at 94°C for 5 minutes; 25 cycles of denaturation at 94°C for 30 seconds, annealing at 55°C for 30 seconds, and extension at 72°C for 60 seconds; followed by a final extension at 72°C for 7 minutes.

As a result, polynucleotides of 528 bp and 509 bp were obtained for fragments H and I, respectively. Using these two fragments as templates, overlapping PCR was performed with primers of SEQ ID NOS: 37 and 40 to obtain a 1010 bp PCR product.

The obtained 1010 bp PCR product was treated with the restriction enzyme Smal (New England Biolabs, Beverly, MA) and then ligated with a pDC24 vector treated with the same restriction enzyme using T4 ligase (New England Biolabs, Beverly, MA). The constructed vector was transformed into *Escherichia coli* DH5α, selected on an LB medium containing kanamycin, and DNA was obtained using a DNA-spin plasmid DNA purification kit (iNtRON). The vector for introducing the A42V mutation into the *ilvN* gene was named pDC24-ilvN(A42V).

**[Table 8]**

| Primer | Base sequence (5' → 3') |
|---|---|
| SEQ ID NO: 37 | cggggatcccccgggAGGACGGTACTCAAATACTAAACTTC |
| SEQ ID NO: 38 | TGCCGAGTGTTTCGGTCTTTACAGACACGAGGGACACG |
| SEQ ID NO: 39 | TGTCTGTAAAGACCGAAACACTCGGCATCAA |
| SEQ ID NO: 40 | cggggatcccccgggGACAACTACATTATTATTATACCACA |

Thereafter, pDC24-ilvN(A42V) was transformed into the wild-type *Corynebacterium glutamicum* ATCC14067 strain by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using SEQ ID NOS: 37 and 40 to amplify a gene fragment, followed by sequence analysis to confirm a strain into which the mutation was introduced. The recombinant strain was named *Corynebacterium glutamicum* CJ7V.

A strain was constructed by transforming *Corynebacterium glutamicum* CJ7V with a vector for deletion of autologous *ilvA* (pDC24_ΔilvA) in the same manner as in Example 1-1, and the strain was named *Corynebacterium glutamicum* CJ7V_ΔilvA.

Meanwhile, a strain was constructed by transforming *Corynebacterium glutamicum* CJ7V with a vector for introducing *Arabidopsis thaliana-derived ilvA* (pDC24_ΔilvA::ilvA(Ath)) in the same manner as in Example 1-2, and the strain was named *Corynebacterium glutamicum* CJ7V_ΔilvA::ilvA(Ath). In order to compare the L-valine-producing ability of the constructed strains, the strains were cultured in the same manner as in Example 2, and the valine-producing ability of each strain was measured and is shown in Table 9 below.

**[Table 9]**

| Strain | Valine-producing ability |
|---|---|
| | g/L/h |
| CJ7V | 0.021 |
| CJ7V_Δilva | 0.006 |
| CJ7V_ΔilvA::ilvA(Ath) | 0.024 |

As shown in Table 9 above, when *ilvA* was deleted, the L-valine-producing ability was only 0.006 g/L/h, whereas it was confirmed that introduction of the *Arabidopsis thaliana*-derived *ilvA* increased the L-valine-producing ability. In addition, it was confirmed that introduction of the *Arabidopsis thaliana-derived ilvA* improved the L-valine-producing ability to a level of 400% relative to the ilvA-deleted strain CJ7V_ΔilvA.

### Example 4: Construction of a leucine-producing strain into which a foreign ilvA(Ath) has been introduced and evaluation of leucine-producing ability

### Example 4-1: Construction of L-leucine-producing strain in which autologous ilvA(Cgl) is deleted

To construct a vector for deletion of a gene (*ilvA,* SEQ ID NO: 43, NCBI accession number "NCgI2046") encoding an autologous threonine dehydratase of *Corynebacterium glutamicum,* PCR was performed using the chromosome of ATCC13032 as a template. The PCR conditions were as follows: an initial denaturation at 95°C for 10 minutes; 28 cycles of denaturation at 95°C for 30 seconds, annealing at 55°C for 40 seconds, and extension at 72°C for 40 seconds; followed by a final extension at 72°C for 5 minutes. As a result, a 1043 bp DNA fragment upstream of the autologous *ilvA* 5' region was obtained using primers of SEQ ID NOS: 44 and 45, and a 1043 bp DNA fragment downstream of the autologous *ilvA* 3' region was obtained using primers of SEQ ID NOS: 46 and 47. The two amplified DNA fragments were purified using a PCR purification kit (PCR Purification Kit, QIAGEN), and the purified amplified products were treated with the restriction enzyme Smal. The treated products were then cloned into a pDC24 vector (SEQ ID NO: 51) heat-treated at 65°C for 20 minutes using an In-Fusion Cloning Kit (TaKaRa) in accordance with the provided manual, thereby constructing pDC24_ΔilvA13032 vector for deletion of autologous *Corynebacterium glutamicum* ilvA.

**[Table 10]**

| Primer | Base sequence (5' → 3') |
|---|---|
| SEQ ID NO: 44 | TGGCCTTCAGCTATGTcccgggCCTACTTTGGCATAGGTTTT |
| SEQ ID NO: 45 | GGTCGACTCTAGAGGATCCCCTGTGTCTCACTCCATCAAC G |
| SEQ ID NO: 46 | GTGAATTCGAGCTCGGTACCCATGATCGCGGCGCCTATTC |
| SEQ ID NO: 47 | CCTATGCCAAAGTAGGcccgggACATAGCTGAAGGCCACCTC |

The constructed pDC24_ΔilvA13032 was transformed by electroporation into *Corynebacterium glutamicum* CJL8109 (WO 2022/163981 A1), which is a leucine-producing strain, to construct strains in which autologous *ilvA* was deleted. The thus-constructed CJL8109 strain in which *ilvA* was deleted was named CJL8126.

### Example 4-2: Construction of L-leucine-producing strain into which Arabidopsis thaliana-derived threonine dehydratase (ilvA(Ath)) was introduced

In order to insert a foreign *ilvA* encoding a threonine dehydratase protein, the autologous *ilvA* gene of *Corynebacterium glutamicum* was used as an insertion site. PCR was performed using the pDC24_ΔilvA::ilvA(Ath) vector prepared in Example 1-2 as a template. The PCR conditions were as follows: an initial denaturation at 95°C for 10 minutes; 28 cycles of denaturation at 95°C for 30 seconds, annealing at 55°C for 30 seconds, and extension at 72°C for 50 seconds; followed by a final extension at 72°C for 5 minutes. As a result, a 1681 bp DNA fragment comprising the *Arabidopsis thaliana-*derived *ilvA* gene (SEQ ID NO: 16) was obtained using primers of SEQ ID NOS: 48 and 49. The amplified product was purified using a PCR purification kit (QIAGEN) and used as an insert DNA fragment for vector construction. The purified amplified product was treated with the restriction enzyme Smal, and cloning was performed using an In-Fusion HD Cloning Kit (Clontech) in accordance with the provided manual, with the molar concentration ratio of heat-treated pDC24_ΔilvA13032 vector at 65°C for 20 minutes to the insert DNA fragment adjusted to 1:2, thereby constructing pDC24_ΔilvA13032::ilvA(Ath), a vector for introducing ilvA(Ath) into the chromosome. The sequences and detailed information of the primers used in the present Example are described in Table 11.

**[Table 11]**

| Primer | Base sequence (5' → 3') |
|---|---|
| SEQ ID NO: 48 | CCTATGCCAAAGTAGGCCCatgTCCATGACCCCGCCTCC |
| SEQ ID NO: 49 | GAGGTGGCCTTCAGCTATGTCCCTTAGTGCATGAGTAGTTT A |
| SEQ ID NO: 50 | GGTGATTGCGAGCAATCCC |

The constructed pDC24_ΔilvA13032::ilvA(Ath) was transformed into *Corynebacterium glutamicum* CJL8109 (WO 2022/163981 A1), a leucine-producing strain, by homologous recombination on the chromosome (van der Rest et al., Appl Microbiol Biotechnol 52:541-545, 1999). Strains in which the vector was inserted into the chromosome by recombination of homologous sequences were selected on a medium containing 25 mg/L kanamycin. Thereafter, the *Corynebacterium glutamicum* transformants in which the second recombination had been completed were subjected to PCR using primers of SEQ ID NOS: 50 and 6, followed by sequence analysis, to confirm whether the autologous *ilvA* ORF was deleted and the *Arabidopsis thaliana-derived ilvA* was introduced at the corresponding position. A strain in which the autologous *ilvA* ORF of the parent strain CJL8109 was replaced with the *Arabidopsis thaliana-derived* ilvA(Ath, Δ1-45, T46M) on the chromosome was named *Corynebacterium glutamicum* CJL8127.

In order to evaluate the leucine-producing ability of the above strains, a flask fermentation titer evaluation was performed.
- Production medium: Glucose 100 g, (NH₄)₂SO₄ 40 g, Soy protein 2.5 g, Corn steep solids 5 g, Urea 3 g, KH₂PO₄ 1 g, MgSO₄·7H₂O 0.5 g, Biotin 100 µg, Thiamine-HCl 1,000 µg, Calcium pantothenate 2,000 µg, Nicotinamide 3,000 µg, and Calcium carbonate 30 g (based on 1 L of distilled water), pH 7.0

Each of the parent strains, *Corynebacterium glutamicum* ATCC13032 and CJL8109, and the constructed CJL8126 and CJL8127 strains was inoculated with one platinum loop into a 250 mL corner-baffle flask containing 25 mL of the production medium, followed by incubation with shaking at 30°C and 200 rpm for 60 hours to produce leucine. After completion of the culturing, the amount of leucine produced was measured by HPLC. The leucine concentrations in the culture broths of each tested strain are shown in Table 12 below.

**[Table 12]**

| Strain | Leucine-producing ability |
|---|---|
| | g/L/h |
| ATCC13032 | 0.02 |
| CJL8109 | 0.06 |
| CJL8126 (CJL8109ΔilvA) | 0.05 |
| CJL8127 (CJL8109ΔilvA::ilvA(Ath)) | 0.13 |

As shown in Table 12 above, it was confirmed that the leucine-producing ability of the ilvA-deleted strain CJL8126 was decreased. In contrast, in the case of the strain CJL8127, which is a leucine-producing strain into which *Arabidopsis thaliana-derived ilvA* was introduced, the leucine-producing ability was improved to a level of 217% relative to the parent strain CJL8109. In addition, it was confirmed that the leucine-producing ability of the *Arabidopsis thaliana-derived* ilvA-introduced strain CJL8127 was improved to a level of 241% relative to the ilvA-deleted strain CJL8126.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

## Claims

1. A microorganism of the genus *Corynebacterium,* into which a threonine dehydratase derived from *Arabidopsis thaliana* is introduced.

2. The microorganism according to claim 1, wherein the threonine dehydratase derived from *Arabidopsis thaliana* comprises an amino acid sequence having at least 75% identity to SEQ ID NO: 15.

3. The microorganism according to claim 1, wherein the threonine dehydratase derived from *Arabidopsis thaliana* is encoded by an *ilvA* gene.

4. The microorganism according to claim 3, wherein the *ilvA* gene derived from *Arabidopsis thaliana* comprises a base sequence having at least 75% identity to SEQ ID NO: 16.

5. The microorganism according to claim 1, wherein the microorganism is one in which endogenous threonine dehydratase activity is further deleted.

6. The microorganism according to claim 1, wherein the microorganism is *Corynebacterium glutamicum.*

7. The microorganism according to claim 1, wherein the microorganism has ability to produce a branched chain amino acid.

8. The microorganism according to claim 7, wherein the branched-chain amino acid is L-valine or L-leucine.

9. The microorganism according to claim 1, wherein the microorganism has increased ability to produce a branched-chain amino acid compared to a non-modified microorganism.

10. A method for producing a branched-chain amino acid, comprising culturing a microorganism of the genus *Corynebacterium,* into which a threonine dehydratase derived from *Arabidopsis thaliana* is introduced, in a medium.

11. The method according to claim 10, further comprising recovering a branched chain amino acid in the cultured microorganism, a culture of the microorganism, a fermentation product of the microorganism, or the medium after cultivation.

12. A method for increasing the production of a branched-chain amino acid, comprising culturing a microorganism of the genus *Corynebacterium,* into which a threonine dehydratase derived from *Arabidopsis thaliana* is introduced, in a medium.

13. A composition for producing a branched-chain amino acid, comprising: a microorganism of the genus *Corynebacterium,* into which a threonine dehydratase derived from *Arabidopsis thaliana* is introduced, a culture of the microorganism, a fermentation product of the microorganism, or a combination of at least two thereof.

14. Use of a microorganism of the genus *Corynebacterium,* into which a threonine dehydratase derived from *Arabidopsis thaliana* is introduced, for producing a branched-chain amino acid.
